# EUROPEAN PATENT SPECIFICATION

(11) **EP 1 503 781 B1**
(45) Date of publication and mention of the grant of the patent: **19.11.2008**
(21) Application number: 03728557.4
(22) Date of filing: 24.04.2003
(51) Int. Cl.: A61K 39/395, A61K 9/00, A23K 1/16, A23L 1/32, A61P 3/06, A61P 3/04, C07K 16/18

(54) **TREATMENT FOR REDUCTION OF BODY WEIGHT GAIN**
BEHANDLUNG ZUR REDUZIERUNG DER ZUNAHME DES KÖRPERGEWICHTS
TRAITEMENT POUR LA REDUCTION DE L'ACCROISSEMENT DU POIDS DU CORPS

(30) Priority: 26.04.2002 US 376166 P; 23.04.2003 US 421393
(43) Date of publication of application: 09.02.2005
(73) Proprietor: PROMEGA CORPORATION, Madison, Wisconsin 53711 (US)
(72) Inventor: STAFFORD, Douglas, C., Madison, WI 53711 (US); KINK, John, A., Madison, WI 53717 (US)
(74) Representative: Glawe, Delfs, Moll
(86) International application number: PCT/US2003/013035
(87) International publication number: WO 2003/090773

(56) References cited:
- EP-A- 1 134 231
- WO-A-01/48221
- WO-A-99/02187
- US-A1- 2003 037 350
- DE LA FOURNIERE L ET AL: "INHIBITORY PROPERTIES AND ANTIGENIC SPECIFICITY OF MONOCLONAL ANTIBODIES TO PANCREATIC COLIPASE" BIOCHIMICA ET BIOPHYSICA ACTA, vol. 998, no. 2, 1989, pages 158-166, XP008057163 ISSN: 0006-3002
- RATHELOT J ET AL: "Studies on the immunological cross-reactivity of various pancreatic colipases. Isolation by immunoaffinity chromatography of a single form of procolipase from porcine pancreas." BIOCHIMICA ET BIOPHYSICA ACTA. 12 JAN 1983, vol. 742, no. 1, 12 January 1983 (1983-01-12), pages 39-48, XP008057162 ISSN: 0006-3002
- RUGANI NATHALIE ET AL: "Immunochemical studies of pancreatic colipase-lipase interaction employing immobilized synthetic peptides" BIOCHEMICAL AND BIOPHYSICAL RESEARCH COMMUNICATIONS, vol. 189, no. 3, 1992, pages 1374-1381, XP008057291 ISSN: 0006-291X
- DEZAN CHRISTINE ET AL: "Monoclonal Antibodies to Human Pancreatic Procolipase: Production and Characterization by Competitive Binding Studies" HYBRIDOMA, vol. 13, no. 6, 1994, pages 509-517, XP008057256 ISSN: 0272-457X
- BOSC-BIERNE I ET AL: "INHIBITION OF PANCREATIC COLIPASE BY ANTIBODIES AND F-A-B FRAGMENTS SELECTIVE EFFECTS OF 2 FRACTIONS OF ANTIBODIES ON THE FUNCTIONAL SITES OF THE COFACTOR" BIOCHIMICA ET BIOPHYSICA ACTA, vol. 827, no. 2, 1985, pages 109-118, XP008057168 ISSN: 0006-3002
- LOWE M E: "STRUCTURE AND FUNCTION OF PANCREATIC LIPASE AND COLIPASE" ANNUAL REVIEW OF NUTRITION, ANNUAL REVIEWS INC., PALO ALTO, CA, US, vol. 17, 1997, pages 141-158, XP009005761 ISSN: 0199-9885
- LOWE MARK E: "Pancreatic triglyceride lipase and colipase: Insights into dietary fat digestion" GASTROENTEROLOGY, vol. 107, no. 5, 1994, pages 1524-1536, XP008057268 ISSN: 0016-5085
- D'AGOSTINO DYMPHNA ET AL: "Decreased postnatal survival and altered body weight regulation in procolipase-deficient mice" JOURNAL OF BIOLOGICAL CHEMISTRY, vol. 277, no. 9, 1 March 2002 (2002-03-01), pages 7170-7177, XP002358552 ISSN: 0021-9258
- DATABASE CAPLUS [Online] MAO ET AL.: 'Protein and cDNA sequences of a 9.02-kilodalton human colipase sequence homolog and their therapeutic uses', XP002967285 Database accession no. 2003:106916 & CN 1 343 711 A 10 April 2002

## Description

### FIELD OF THE INVENTION

The present invention is defined in the claims and describes a therapy for reducing body weight gain humans or other animals using antibodies that interfere with macromolecular nutrient digestion. Certain enzymes and enzyme co-factors found in the digestive tracts of animals are essential for nutrient breakdown prior to absorption and use as an energy source. The present invention describes the use of antibodies that interfere with the digestion of dietary lipids to promote weight loss or impede weight gain.

### BACKGROUND OF THE INVENTION

Obesity is now an epidemic in the United States. Approximately 97 million adults in the United States are either overweight or obese, with the prevalence increasing markedly over the last decade (Kucamarski RJ et al. Obes. Res. 1997; 5:542-548). Moreover, almost 50% of the population in developed countries such as the United States are 22% above their recommended weight (Flegal KM et al. Int. J.Obes Relat. Metab. Disord 1998;2 2:39-47). Obesity is second only to smoking as a cause of preventable death in the United States (McGinnis J, et al. JAMA 1993;270:2207-2212). A study in 1991 estimated the number of overall annual deaths attributed to obesity in the United States to be 2 80,000 (Allison DS, et al. JAMA 1999;282:1530-1538). Obesity and hyperlipidemia are conditions associated with a high rate of morbidity and early mortality (Martin MJ, et al. Lancet 1986;2:933-936, Van Itallie TB, Ann. Intern. Med. 1985;103:983-988). Obesity primarily caused by high fat intakes is associated cardiovascular disease, type 2 diabetes, osteoarthritis, hypertension and cancer (National Institutes of Health, National Heart, Lung, and Blood Institute. US Dept. of Health and Human Services, public Health Service, NIH, NHLBI;1998). Dietary fat, primarily as triacylglycerides contributes a high percentage, from 30-44 of total calories consumed by people in western society (Kromhout D, Am. J. Clin. Nutr. 1983;37:295-299).

Current dietary guidelines recommend reductions in fat consumption to 30% or less (Nation Research Council. Diet and Health. Washington DC:Natl Acad. Press, 1989). Because compliance with low-fat diets has been problematic, pharmacological therapies are often added to the dietary therapy. About 58 million Americans spend about $30 billion annually for these pharmacological therapies to control obesity.

Obesity is generally a chronic disease that requires long-term behavior modification supplemented with drug therapy. Diet-drug therapies have shown moderate benefits at best though are poorly tolerated by patients (Jones SP, et al. Int. J. Obes. 1995; 19 (suppl 2):41. Abstract 071). Current weight loss therapies primarily function systemically to alter neurotransmitters to suppress appetite. While most therapies have been moderately effective, many have systemic side effects.

Sibutramine (Meridia), a recently approved drug controls appetite by modifying levels of the neurotransmitter norepinephrine producing a chemically induced illusion of satiety. However, this drug and drugs in this class have a variety of central nervous system (CNS) aide-effects, including headache, anxiety, fatigue, insomnia and hypertension. Some of these appetite-suppressing drugs have much more serious side-effects. Several drugs such as dexfenfluramine (Redux) or fenfluramine/phenteramine (Fen-phen), which act on the neurotransmitter serotonin, have been withdrawn from the market by the United States Food and Drug Administration in 1997. Both drugs caused life-threatening primary pulmonary hypertension, valvular heart dysfunction and neurotoxicity. While the safer diet drugs, such as phenylpropanpamine (Dexatrim) have more moderate side effects, none of the current therapies work by maximizing the effectiveness of reducing fat consumption through dieting. Despite recent advances in treatment, there remains a significant need for a safe, non-systemic therapy which works in concert with the standard first-line dietary approach.

New strategies are being developed to reduce dietary fat uptake in combination with standard dietary therapy. The use of fat substitutes such as Olestra, composed of indigestible long-chain fatty acids, is one approach along these lines. Unfortunately, Olestra has been met with a less than enthusiastic response by consumers. In addition to side-effects due to malabsorption, these fat substitutes can also severely reduce the uptake of fat-soluble vitamins such as vitamin K.

Drugs that selectively limit the intestinal absorption of dietary fat, in addition to that controlled by dietary manipulation alone, would be a useful therapeutic agent for the treatment of obesity. Ideally, these drugs would be effective with minimal side-effects thus promoting wide-spread compliance. A newer approach being explored is the use of inhibitors to gastrointestinal enzymes involved in fat metabolism. These enzymes are particularly attractive targets because they are found within the lumen of the GI tract, and the inhibitor need not get into circulation to be effective. This luminal route of action tends to limit the adverse effects associated with systemic therapies.

Triacylglyceride lipases are ubiquitous enzymes required for all aspects of fat metabolism. Found in the gastrointestinal tract, these enzymes mediate the digestion of triglycerides and their uptake into tissues. Triacylglycerides contribute to greater than 95% of the dietary fats in the western diet (Patton JS , et al. Am. J. Physiol. 1981;241:G328-336). Lipases hydrolyze the ester bonds in the triacylglycerides releasing free fatty acids and mono-acylglycerols. Dietary triacylglycerides must be cleaved into free fatty acids and monoacylglycerols before they are absorbed by intestinal enterocytes. In the absence of lipases, dietary triacylglycerides are not absorbed and pass into the stool. Consequently, reduced absorption of digested triacylglycerides should result in weight loss.

One new drug, Xenical (Orlistat, Hoffmann-La Roche), has been recently approved by the FDA for treatment of obesity. This drug is a potent inhibitor of pancreatic lipase; secreted into the proximal small intestine by acinar cells of the pancreas (Borgstrom R, Gastroenterol. 1984;86:194-196). Xenical is a synthesized derivative of tetrahydrolipstatin a naturally occurring lipase inhibitor produced by *Streptomyces toxytricini.* It has been shown to be effective at reducing fat absorption in humans and animals (Hogan SA, et al. Int J. Obes. 1987, 3 (suppl 11):35-42 1987, Hauptman JB, et al. Am. J. Clin. Nutr. 1992;55 (suppl 1): 309S-313S). Xenical is minimally absorbed and prevents the absorption of about 30% of dietary fat consumed. In clinical trials of patients on a low-fat diet, those taking Orlistat lost an average of 3-4% of their body weight in a year compared with placebo-treated controls. Unfortunately, in a 1-year trial of maintenance therapy, 76% of patients taking Orlistat regained weight compared with 84% of the placebo group. Side-effects were seen in 20-40% of the patients which included flatulence with discharge, oily spotting, and malabsorption of fat-soluble vitamins and beta-carotene. While these side effects can be unpleasant, they *do not* preclude the use of the drug in most patients. Overall, clinical trails indicate that Xenical is modestly effective with some unpleasant side-effects.

Antibodies to lipase have also been used to impair dietary lipid metabolism (Pimental, J., International Patent Application, WO 99/02187). In rodent studies, lipase antibodies were shown to increase the amount of animal ration needed to gain 1 gram of body weight. However, the antibodies were not shown to be able to reduce weight or impede weight gain.

### DEFINITIONS

To facilitate understanding of the invention, a number of terms are defined below.

As used herein, the term "neutralizing" is used in reference to antagonists, which may include antibodies or antibody preparations, that interfere with enzymes or enzyme co-factors which are able to reduce(in some cases substantially reduce) a specific enzymatic activity. Complete elimination of activity is not required. For certain embodiments, it is sufficient that there is a reduction in enzymatic activity (for example, in certain embodiments, it is sufficient that there is approximately a 50% reduction, more preferably approximately a 75% reduction, still more preferably a reduction of approximately 90% or more).

As used herein, the term "purified" or "to purify" refers to at least the partial removal of one or more contaminants from a sample. For example, antibodies may be purified by at least partial removal (for example, approximately a 20% removal, more preferably approximately a 50% removal, still more preferably a reduction of approximately 90% or more) of contaminating non-immunoglobulin proteins. The removal of non-immunoglobulin proteins results in an increase in the percent of specific target-reactive immunoglobulins in the antibody preparation. While the present invention is not limited to the manner of purification, one method for purification of antibodies from egg yolks is by extraction and precipitation using polyethylene glycol.

The term "subject" when used in reference to administration of compositions comprising antagonists to colipase refers to the recipient animal to whom said antagonists are administered. The subject may be an animal, including mammals and more particularly, humans, in whom it is desirable to reduce bodily weight or impede bodily weight gain. In the case where the subject is a human, the present invention is not limited to only humans that are patients; indeed; in certain embodiments, it is contemplated that treatment can be accomplished without hospitalization.

The term "antibody" includes antibody fragments such as single chain antibodies, Fab fragments, Fab₂ fragments and other fragments which are capable of binding colipase. It is not intended that the present invention be limited by the host used to make antibodies. Moreover, in certain embodiments, the antibody can be made recombinantly.

The term "antagonist to colipase" includes antibodies. However, it is contemplated that antagonists can be fashioned from colipase as well as the receptor for colipase. For example, small peptides or mimetics can be made based on sequences of colipase; such peptides can be variant in amino acid sequence (one or two different amino acids) or wild type. Such peptides can be protected from breakdown by protecting groups on the N-terminus and C-terminus.

In one embodiment, the present invention contemplates formulations which "protect the antibody from digestive agents." Typically, such formulations are solid formulations comprising coatings which shield the antibody from enzymatic breakdown. Complete protection is not required.

It is sufficient that some protection is afforded (for example, approximately a 20% reduction in antibody breakdown, more preferably approximately a 50% reduction, still more preferably a reduction of approximately 90% or more).

### SUMMARY OF THE INVENTION

The present invention is defined in the claims and provides compositions comprising an antagonist to colipase. Colipase is another key protein involved in fat metabolism. This protein is an essential enzyme cofactor of pancreatic lipase. Colipase stabilizes the active conformation of lipase by binding to the carboxy-terminal, non-catalytic domain of lipase (Lowe M, Ann. Rev. Nutr. 1997;17:141-158). This enzyme complex allows the hydrolysis of the dietary triacylglyceride at the lipid-water interface.

Importantly, the interaction of pancreatic lipase and colipase is needed for activity. Pancreatic lipase is inhibited by physiological concentrations of bile salts as well as by phospholipids and proteins present in emulsions of dietary lipids. Colipase restores activity to pancreatic lipase and permits efficient digestion of dietary fats under these inhibitory conditions (Lowe M, Ann. Rev. Nutr. 1997;17:141-158). The essential role of colipase in triacylglyceride digestion makes it an attractive enzyme target for drug therapy to control fat absorption.

The present invention relates to the use of orally-delivered anti-colipase antibodies for the manufacture of a medicament for therapeutically reducing body weight gain by reducing dietary fat metabolism and absorption in humans as well as other animals. The examples of the present invention demonstrate the production of neutralizing antibodies to pancreatic lipase and colipase by hyper-immunizing egg-laying hens. Both antibodies were tested in rats, showing the relative ability of these antibodies to effect impedance of body weight gain.

Such antagonist is capable of reducing lipase enzyme activity that occurs normally in the lumen of the intestinal tract. The antagonist is an antibody that binds to colipase. In a further embodiment, colipase antibodies are produced in laying hens. In one embodiment, it is intended that the antagonist of the present invention be formulated as a medicament.

The antibodies of the present invention find use in humans or other animals as a medicament to therapeutically reduce body weight gain. In a one embodiment, the medicament would be in the form of a solid dosage form, such as a tablet or capsule, suitable for oral administration. In another embodiment, the medicament would be admixed with a foodstuff intended for consumption. In a further embodiment, the antibodies would be protected by pharmaceutical excipients and/or chemical coatings to afford protection from digestive agents.

In yet another embodiment, the dosage would be in the form of a powder that could be mixed with potable liquids and administered orally. Such potable liquids may contain compounds to aid in survival of the antagonist while passing through the digestive tract of the subject.

In one embodiment, the invention contemplates the use of a preparation comprising an antibody to colipase effective in impeding body weight gain for the manufacture of a medicament for therapeutically reducing body weight gain.

In another embodiment, the medicament is combined with a lipid rich diet.

In yet another embodiment, said medicament is for oral administration.

The present invention also contemplates compositions. In one embodiment, the present invention contemplates a preparation comprising an antagonist to colipase (including but not limited to a polyclonal antibody).

### DESCRIPTION OF THE INVENTION

The present invention is defined is the claims and is directed to: i) preparations comprising one or more antagonists to colipase, and; ii) use of such an antagonist, as a medicament in a variety of forms, to modulate bodily weight. Because lipids represent a large portion of the nutritional and caloric content of a typical diet, interfering with lipid breakdown and absorption would be of considerable use in impeding weight gain or promoting weight loss.
i) Compositions of antagonists to colipase. The present invention contemplates an antagonist to colipase capable of interfering with the hydrolytic activity of one or more gastrointestinal lipases. To show that antagonism of colipase is effective in reducing lipase activity, antibodies directed to colipase were used. Colipase antibodies were produced in laying hens following immunization with colipase protein. In a preferred embodiment, the antagonist is an antibody. In yet another embodiment, the antibody is harvested from the chicken eggs. It is also contemplated that antagonists may be in the form of antibody fragments.
   Antibodies of the present invention may be produced a variety of animal species and have a variety of compositions. For example, antibodies may be produced following immunization of an animal with the entire colipase protein, a portion of the colipase protein, peptides representing regions or epitopes of the colipase protein, or other molecules that are capable of inducing antibodies reactive to colipase. Antibodies resulting from immunization of an animal would normally be polyclonal, that is, containing multiple antibody specificities reacting to colipase with varying affinities. The present invention contemplates the use of polyclonal antibodies because of their ease and low cost of production. A variety of animal species could be used to produce polyclonal antibodies of the present invention. For example, large mammals such as horses, cows, goats, sheep, and the like, can produce large amounts of antibodies that can be harvested from their blood and their milk. Alternatively, avian species could be used and the antibodies harvested from their eggs.
   However, the present invention is not limited to the production of polyclonal antibodies following immunization of animals. For example, monoclonal colipase antibodies having a single specificity (or a mixture of two or more monoclonal antibodies) are also contemplated. Monoclonal antibodies may be produced from hybridoma cell lines placed in living animals (production of acites fluid) or in bioreactors (production of culture supernatants). Monoclonal antibodies, or fragments of whole *antibodies, may also* be produced recombinantly using animal, bacterial, yeast, insect, or plant expression systems.
   Antibodies of the present invention may be used in a plurality of forms. It is contemplated that useful antibodies of the present invention may be in a crude (that is, unpurified) form as might be found in animal serum, eggs, cell culture supernatant, or plant tissues. In a preferred embodiment, the antibodies would be purified (through separation, fractionation, precipitation, chromatography, absorption, filtration, and the like) to enrich the concentration of the desired antibody protein in relation to non-antibody substances. In one embodiment, antibodies, whether crude or purified, may be mixed with excipients that function to enhance chemical stability, prevent aggregation, allow for compaction, or otherwise enable the antibody protein to be taken by a subject by mouth and be effective within the lumen of the gastrointestinal tract. In addition to excipients, the present invention contemplates the use of enteric coatings to protect the antibody protein from digestive agents within the gastrointestinal tract. In preferred embodiments, the antibody would be processed in the form of a capsule, microcapsule, tablet, suspension, powder, or solution, each of which would be orally deliverable. In yet another embodiment, the antibodies could be admixed with a foodstuff intended for consumption.
ii) Use of colipase antagonists to modulate bodily weight. The present invention contemplates the use of colipase antagonists as a medicament to reduce body weight gain by interference with normal intestinal lipid metabolism. Modulation of bodily weight could include reduction of weight or impedance of weight gain. It is contemplated that the medicament would be useful in humans, or other animals, where bodily weight modulation is desirable.
   The medicament of the present invention could have various modes of delivery. In a preferred embodiment, the medicament of the present invention is in a solid dosage form that can be taken by mouth. In another embodiment, the medicament is mixed with a potable liquid suitable for drinking. In yet another embodiment, the medicament is admixed with a foodstuff suitable for consumption.
   The following examples serve to illustrate certain preferred embodiments and aspects of the present invention and are not to be construed as limiting the scope thereof.
   In the disclosure that follows, various abbreviations are used: °C (degrees Centigrade); g (the force of gravity on Earth); mg (milligram); ug (microgram); ml (milliliter); ul (microliter) PBS (phosphate buffered saline); PEG (polyethyleneglycol); IgY (immunoglobulin of yolk); nm (nanometer); EIA (enzyme immunoassay); U/L (units per liter, and; SEM (standard error of the mean).

### EXAMPLE 1

### Production of antibodies to pancreatic lipase and colipase in the hen

This example involved (a) preparation of the immunogen and immunization of chickens, (b) purification of pancreatic lipase antibodies and colipase antibodies (IgY) from egg yolk, and (c) detection of specific antibodies in the purified IgY preparations.

### (a) Preparation of the immunogen and immunization.

Human pancreatic lipase (catalog number 437709) was purchased from Calbiochem Corp, La Jolla, CA. Porcine colipase (catalog number 142A4000) was purchased from Calzyme Laboratories, San Luis Obispo, CA. The colipase, which was supplied as a freeze-dried powder was reconstituted in phosphate-buffered saline (PBS) pH 7.0-7.4 at 10 mg/ml. Egg-laying Leghorn hens were immunized with either approximately 10 ug of pancreatic lipase or 250 ug of colipase each mixed with 75 ug Qui1 A adjuvant (Superfos Biosector, Denmark, distributed by Accurate Chem., Westbury, N.Y.) in PBS. The immunization volume was 0.5 ml and delivered via one sub-cutaneous or intramuscular injection. The hens were immunized at least 3 times every 2-4 weeks.

### (b) Purification of pancreatic lipase IgY and colipase IgY from egg yolks.

Eggs were collected from hens given either pancreatic lipase or colipase immunizations at least 3-5 days after receipt of the last booster immunization. Egg yolk immunoglobulin (IgY) was extracted using a two-step polyethylene glycol (PEG)8000 method performed according to a modification of the procedure of Polson (Poulson et al. Immunol. Comm. 1980; 9:495). The yolks were separated from the whites and the yolks were placed in a graduated cylinder. The pooled yolks were blended with 4 volumes of PBS and PEG was added to a concentration of 3.5%. When the PEG was dissolved, the protein and lipid precipitate that formed was removed by centrifugation at 9,000 x g for 15 minutes. The supernatant was decanted and filtered through 4 layers of gauze to remove the floating particulates and a second PEG step was performed by adding PEG to a final concentration of 12% (the supernatant was assumed to contain 3.5% PEG). After a second centrifugation, the supernatant was discarded and the IgY pellet resuspended in PBS at approximately 1/6 the original yolk volume. IgY extracted from the eggs of immunized hens are designated as "pancreatic lipase" or "colipase IgY" while IgY extracted from the eggs of unimmunized hens is designated "preimmune IgY." The concentration of the fractionated IgY was estimated by measuring absorbance at 280nm (an optical density at 280 nm of 1.3 equals 1 mg of IgY/ml). The protein concentration of the IgY preparations were between 20-35 mg/ml.

### (c) Detection of pancreatic lipase and colipase antibody activity in the IgY preparations.

In order to determine if an antibody response was generated against pancreatic lipase or colipase and to determine relative levels of the responses, enzyme-linked immunosorbant assays (EIA) were performed. Briefly, ninety-six well Maxisorb (Nunc) micro-titer plates were coated overnight at 4°C with 100 ul/well with either pancreatic lipase or colipase at 2.5 ug/ml in PBS. The wells are then blocked with PBS containing 1% BSA and 0.05% Tween 20 and incubated for about 1 hour at 37°C. The blocking solution was removed. The immune or preimmune IgY was diluted in PBS containing BSA and the plates were incubated for 1 hour at 37°C. The plates were washed 3 times with PBS containing 0.05% Tween 20 and three times with PBS alone. Alkaline phosphatase-conjugated anti-chicken IgG was diluted 1:1000 in PBS containing 1% BSA and 0.05% Tween 20, added to the plates and incubated 1 hour at 37°C. The plates were washed as above and p-nitrophenyl phosphate at 1 mg/ml in 0.05 M Na₂CO₃, pH 9.5, 10 mM MgCl₂ was added. The absorbance at 410 nm of the solution in each well was determined using a Dynatech plate reader about 30 minutes after substrate addition.

The EIA results indicated that antibody activity could be readily measured in both the pancreatic lipase IqY and colipase IgY preparations. EIA titer is a convenient measure of antibody activity. EIA titer is defined as the reciprocal of the highest immune IgY dilution generating an absorbance signal that is about 3-fold higher than that of preimmune IgY control sample. EIA titers to both pancreatic lipase and colipase were at least 10,000. These results indicated hens readily respond to pancreatic lipase and colipase by producing antibodies. None of the hens receiving an immunization exhibited an adverse reaction.

### EXAMPLE 2

### Demonstration of Pancreatic Lipase IgY Neutralizing Ability Using an in vitro Enzyme Assay.

In this example, the pancreatic lipase IgY was evaluated in an in vitro assay to determine if pancreatic lipase enzyme activity could be neutralized by antibodies in the IgY preparation. The effectiveness of pancreatic lipase IgY at neutralizing the enzymatic activity of pancreatic lipase was determined using a commercially-available in vitro assay (Lipase-PS assay catalog number 805-A, Sigma Chemical Co., St. Louis, MO). This assay was designed to measure serum lipase activity which is widely used for the diagnosis of acute pancreatitis. The assay is a colorimetric method in-which 1,2-diglyceride is hydrolyzed to 2-monoglyceride and fatty acid. This assay was modified for our purposes to determine if pancreatic lipase IgY could inhibit the pancreatic lipase activity in the presence of substrate. The Lipase PS assay was also modified to conform to a microtiter plate assay format.

Different concentrations of pancreatic lipase IgY or preimmune IgY as a control were diluted in phosphate-buffered saline (PBS) and 100 ul was added in duplicate to a 95 well-microtiter plate (Nunc, Maxisorb). Another set of controls consisted of two sets of wells containing 100 ul of only PBS to serve as a blank and PBS plus the lipase standard to serve as a positive control. To each well, 100 ul of Lipase-PS assay kit substrate reagent diluted in substrate diluent buffer was added. The reconstituted substrate reagent contains lipase substrate (1,2 diglyceride), cofactors (porcine colipase, 1340 U/L) and substrates for the chromogenic reaction. The chromogenic reaction which occurs when active lipase is present in the sample is directly proportional to pancreatic lipase activity. Human pancreatic lipase PS enzyme standard (1.5 ul/well)(supplied in the assay kit) at 258 U/L was added to each well except the blank PBS wells, followed by incubation for 5 minutes at 37°C. Lastly, the Lipase-PS activator reagent (30 ul) containing bile salts, was added to each well to initiate the enzymatic reaction. The microtiter plate was incubated at 37°C in a ELx808 plate reader, (Bio-TeK Instruments Inc, Winooski, VT). The absorbance of each well at 550 nm was read after 3 minutes of incubation at 37°C (initial A) and then again 2 minutes later (final A). Lipase activity in U/L was determined by calculating the change in absorbance of the test sample minus the blank divided the change in the absorbance of standard control minus the blank multiplied by the labeled activity (258 U/L) of the standard.

The assay results showed that anti-pancreatic lipase IgY neutralized the activity of pancreatic lipase. Concentrations of anti-pancreatic lipase at 0.04 mg/ml to 0.66 mg/ml inhibited 80-90% of the standard lipase activity. In contrast, equivalent concentrations preimmune IgY failed to neutralize any of the standard enzyme activity. Higher IgY concentrations of both the preimmune or the pancreatic lipase IgY nonspecifically inhibited lipase activity in this assay. The results demonstrate that the use chicken antibodies against pancreatic lipase is an effective method to inhibit pancreatic lipase activity.

### EXAMPLE 3

### Demonstration of Colipase IgY Neutralizing Ability Using an in vitro Enzyme Assay

This example involved the testing of the colipase IgY neutralizing ability in an *in vitro* enzyme assay. The ability of colipase IgY to neutralize the bioactivity of colipase was determined by using the Lipase-PS assay for lipase activity described in Example 2. Since colipase is an obligatory enzyme cofactor of pancreatic lipase in the presence of bile salts, the inhibition of colipase should prevent the enzymatic reactions involved in the Lipase -PS assay. Therefore, the inhibition of colipase was measured indirectly by determining the effect of colipase IgY on the enzymatic activity in the lipase assay.

The Lipase-PS assay using colipase IgY was performed essentially as described in Example 2. Different concentrations of colipase or preimmune IgY diluted in PBS were preincubated at 37°C with the lipase substrate reagent. The lipase substrate reagent contained a fixed amount of porcine colipase at 1340 U/L. The human lipase enzyme standard and activator were then added as the assay proceeded as described above. The wells are read at 550 nm after 3 and 5 minutes of incubation at 37°C. Pancreatic lipase activity was calculated as described in Example 2.

The assay results indicated that colipase IgY was effective at inhibiting the hydrolysis of diglyceride in the lipase assay. Concentrations of colipase IgY at 0.17 mg/ml and 0.04 mg/ml inhibited 49% and 29% of the standard lipase activity. Since colipase was fixed component within Lipase-PS substrate buffer, improving the inhibitory effect by colipase IgY by modifying the amount of colipase within the reaction was not possible. Importantly however, preimmune IgY at equivalent concentrations was unable to inhibit any lipase activity. At higher concentrations of IgY, lipase activity was nonspecifically inhibited in this assay. The results indicate that the colipase IgY could inhibit colipase activity and that this antibody is an effective way to inhibit this enzymatic pathway.

### EXAMPLE 4

### The Reduction of Body Weight Gain in Sprague-Dawley Rats Using Oral Pancreatic Lipase IgY or Colipase IgY

This example involved the testing the effect of pancreatic lipase IgY and colipase IgY in an animal model of diet-induced obesity. This example illustrates that the inhibition of colipase in vivo using orally administered colipase IgY can prevent weight gain in rats fed a high fat diet. Pancreatic lipase IgY treatment also had a modest effect on weight gain in this model.

Diet-induced obesity was studied in rats using a model substantially as described by Ackroff, K. using Sprague-Dawley rats (Ackroff K, et al. Am. J. Physiol. 1996; 271:R48-R54). Rats that overeat and become obese when given palatable food serve as a good model for dietary obesity in man (Schemmel RO, et al. J. Nutr. 1970;100:1041-1048). Four groups ("Group" 1-4) containing 6-7, 6 week-old male rats (Charles River, Wilmington, MA) were all given a standard balanced low-fat rodent chow (Formulab 5008, PMI Feeds, Inc, Richmond, IN) ad libitum. Rats in Group 1 were fed only the rodent chow and served as an untreated control. In addition to rodent chow, the diets of the rats in Groups 2, 3, and 4 were supplemented with egg yolks as a high-fat, dietary component. Approximately 35% of egg yolk by weight consists of fat. Rats in Groups 2, 3, and 4 were given a choice between a fat (egg yolk) and moderately low fat (rat chow) diet. Rats in Group 2 were fed egg yolks from eggs harvested from preimmune hens. Rats in Groups 3 and 4 were fed egg yolk from eggs of the hens immunized with either pancreatic lipase IgY or colipase IgY. Egg yolks were harvested and pooled in a clear plastic animal water bottles with volume graduations. The yolks were then mixed as a suspension consisting of 2 parts yolk to 1 part 0.3% sodium benzoate in water (final sodium benzoate concentration of 0.1%). The dilution of the egg yolks with a solution of sodium benzoate minimized microbial contamination and permitted the flow of yolks through the water bottle sipper.

During the study, the rats consumed the egg yolks mixture preferentially over the balanced rat chow. Daily egg yolk consumption in each group was very comparable and consisted of about 200 ml of yolk or about 12 eggs/day. Rats from each group were weighed approximately once per week to compare the rate of weight gain between the 4 groups. The average weight with standard error (SEM) for each group during treatment is shown in Table 1.

**Table 1 Mean Body Weight* of Rats**

| Group | Day 0 | Day 16 | Day 27 | Day 35 | Day 50 |
|---|---|---|---|---|---|
| | | | | | |
| Untreated Control | 165(2) | 313(6) | 365(11) | 409(17) | 458(19) |
| | | | | | |
| Preimmune IgY | 167(2) | 337(7) | 418(7) | 466(8) | 533(6) |
| | | | | | |
| Pancreatic lipase IgY | 168(1) | 344(7) | 404(11) | 452(13) | 504(16) |
| | | | | | |
| Colipase IgY | 167(1) | 326(9) | 363(14) | 404(15) | 450(16) |

| | | | | | |
|---|---|---|---|---|---|
| * Mean weights are shown in grams and the standard errors of the mean are shown in grams (in parenthesis). | | | | | |

The experimental results indicated that colipase IgY could significantly impede weight gain in the rat obesity model compared to the preimmune IgY treated rats. Using the Mann Whitney U-test, p values of ≤ 0.05 are considered significant. Mean rat weights in the colipase IgY treated group began to differ significantly from the preimmune IgY treated rats at day 27 (p=0.0025). The mean weight differences between these two groups remained significant throughout the treatment period. Remarkably, the mean weight gain of the colipase IgY treated rats was not significantly different at any time in the treatment period from the weight gain of the untreated control animals that were not given supplemental dietary fat.

After 50 days of treatment, the statistical differences in mean weight between the preimmune IgY and colipase IgY treated rats increased. The mean weight of the colipase IgY treated rats were 16%, or 83 grams, less than the preimmune IgY treated rats (p=0.001). Moreover, at that time, every member treated with colipase IgY weighed less than any rat in the preimmune IgY treated group. For example, the lightest member in the preimmune IgY treated group (510 g) still weighed 11 grams more than the heaviest colipase IgY treated member (499 g).

The experimental results also indicated that colipase IgY was more effective than pancreatic lipase IgY at impeding weight gain in the rat obesity model. After 35 days of treatment, the rats in the colipase IgY treated groups weighed significantly less than the pancreatic lipase IgY treated group (p=0.025). After 50 days of colipase IgY treatment, the weights of the rats in that group remained significantly lower than the pancreatic lipase IgY treated rats (p<0.05).

Rats treated with pancreatic lipase IgY did not show a statistically significant difference in weight when compared to the preimmune IgY treated controls at any of the measurement points. For example, at day 50 the calculated p value between those treatment groups was <0.1.

The overall results in this study indicated that oral colipase IgY treatment could significantly reduce weight gain in rats fed a high-fat diet. These results indicate that oral colipase IgY therapy is an effective method to impede weight gain in animals consuming a fat-rich diet. These results also show that antibodies directed to colipase are more effective than similar antibodies to pancreatic lipase and that the effectiveness increases over the treatment period (thus, treatment periods for humans of 30 days or more are preferred). While not significant at the p≤0.05 level, the pancreatic lipase IgY treated animals did show reduced mean weight as compared to the preimmune IgY treated animals.

From the forgoing is should be clear that the present invention provides compositions, and uses for an effective medicament comprising an antibody to colipase.

## Claims

1. A preparation comprising an antibody to colipase effective in impeding body weight gain for use in therapeutically reducing body weight gain.

2. The preparation of Claim 1, wherein said preparation is for oral administration.

3. The preparation of Claim 2, wherein said oral formulation is a capsule, microcapsule, tablet, powder, suspension, or solution.

4. The preparation of Claim 2, wherein said oral formulation protects said antibody from digestive agents.

5. The preparation of Claim 1, wherein said antibody is produced in an animal.

6. The preparation of Claim 5, wherein said antibody is produced in a mammal.

7. The preparation of Claim 5, wherein said antibody is produced in an avian.

8. The preparation of Claim 5, wherein said antibody is produced recombinantly.

9. The preparation of Claim 1, wherein said antibody is unpurified.

10. The preparation of Claim 1, wherein said antibody is purified.

11. Use of a preparation comprising an antibody to colipase effective in impeding body weight gain for the manufacture of a medicament for therapeutically reducing body weight gain.

12. The use of Claim 11, wherein said medicament is combined with a lipid rich diet.

13. The use of Claim 11, wherein said medicament is for oral administration.

14. The use of Claim 13, wherein said oral formulation is a capsule, microcapsule, tablet, powder, suspension, or solution.

15. The use of Claim 13, wherein said oral formulation protects said antibody from digestive agents.

16. The use of Claim 11, wherein said antibody is produced in an animal.

17. The use of Claim 16, wherein said antibody is produced in a mammal.

18. The use of Claim 16, wherein said antibody is produced in an avian.

19. The use of Claim 16, wherein said antibody is produced recombinantly.

20. The use of Claim 11, wherein said antibody is unpurified.

21. The use of Claim 11, wherein said antibody is purified.

## Patentansprüche

1. Präparat umfassend einen Antikörper gegen Colipase, der effektiv ist in dem Verhindern der Körpergewichtszunahme zur Verwendung in der therapeutischen Reduktion von Körpergewichtszunahme.

2. Präparat nach Anspruch 1, worin das Präparat für die orale Administration ist.

3. Präparat nach Anspruch 2, worin die orale Formulierung eine Kapsel, Mikrokapsel, Tablette, Puder, Suspension oder Lösung ist.

4. Präparat nach Anspruch 2, worin die orale Formulierung den Antikörper vor verdauenden Agenzien beschützt.

5. Präparat nach Anspruch 1, worin der Antikörper in einem Tier produziert ist.

6. Präparat nach Anspruch 5, worin der Antikörper in einem Säugetier produziert ist.

7. Präparat nach Anspruch 5, worin der Antikörper in einem Vogel produziert ist.

8. Präparat nach Anspruch 5, worin der Antikörper rekombinant produziert ist.

9. Präparat nach Anspruch 1, worin der Antikörper ungereinigt ist.

10. Präparat nach Anspruch 1, worin der Antikörper gereinigt ist.

11. Verwendung eines Präparats umfassend einen Antikörper gegen Colipase, der effektiv ist in der Verhinderung der Körpergewichtszunahme, zur Herstellung eines Medikaments für die therapeutische Reduktion der Körpergewichtszunahme.

12. Verwendung nach Anspruch 11, worin das Medikament mit einer lipid-reichen Diät kombiniert wird.

13. Verwendung nach Anspruch 11, worin das Medikament für die orale Verabreichung ist.

14. Verwendung nach Anspruch 13, worin die orale Formulierung eine Kapsel, Mikrokapsel, Tablette, Puder, Suspension oder Lösung ist.

15. Verwendung nach Anspruch 13, worin die orale Formulierung den Antikörper vor verdauenden Agenzien beschützt.

16. Verwendung nach Anspruch 11, worin der Antikörper in einem Tier produziert ist.

17. Verwendung nach Anspruch 16, worin der Antikörper in einem Säugetier produziert ist.

18. Verwendung nach Anspruch 16, worin der Antikörper in einem Vogel produziert ist.

19. Verwendung nach Anspruch 16, worin der Antikörper rekombinant produziert ist.

20. Verwendung nach Anspruch 11, worin der Antikörper ungereinigt ist.

21. Verwendung nach Anspruch 11, worin der Antikörper gereinigt ist.

## Revendications

1. Préparation comprenant un anticorps de la colipase efficace pour ralentir la prise de poids corporel pour une utilisation dans la réduction thérapeutique de gain de poids corporel.

2. Préparation selon la revendication 1, dans laquelle ladite préparation est destinée à une administration orale.

3. Préparation selon la revendication 2, dans laquelle ladite formulation orale est une capsule, une microcapsule, un comprimé, une poudre, une suspension ou une solution.

4. Préparation selon la revendication 2, dans laquelle ladite formulation orale protège ledit anticorps des agents digestifs.

5. Préparation selon la revendication 1, dans laquelle ledit anticorps est produit chez un animal.

6. Préparation selon la revendication 5, dans laquelle ledit anticorps est produit chez un mammifère.

7. Préparation selon la revendication 5, dans laquelle ledit anticorps est produit chez un oiseau.

8. Préparation selon la revendication 5, dans laquelle ledit anticorps est produit par recombinaison.

9. Préparation selon la revendication 1, dans laquelle ledit anticorps est non purifié.

10. Préparation selon la revendication 1, dans laquelle ledit anticorps est purifié.

11. Utilisation d'une préparation comprenant un anticorps de la colipase efficace pour réduire le gain de poids corporel pour la fabrication d'un médicament pour réduire thérapeutiquement le gain de poids corporel.

12. Utilisation selon la revendication 11, dans laquelle ledit médicament est combiné avec un régime riche en lipides.

13. Utilisation selon la revendication 11, dans laquelle ledit médicament est destiné à une administration orale.

14. Utilisation selon la revendication 13, dans laquelle ladite formulation orale est une capsule, une microcapsule, un comprimé, une poudre, une suspension ou une solution.

15. Utilisation selon la revendication 13, dans laquelle ladite formulation orale protège ledit anticorps des agents digestifs.

16. Utilisation selon la revendication 11, dans laquelle ledit anticorps est produit chez un animal.

17. Utilisation selon la revendication 16, dans laquelle ledit anticorps est produit chez un mammifère.

18. Utilisation selon la revendication 16, dans laquelle ledit anticorps est produit chez un oiseau.

19. Utilisation selon la revendication 16, dans laquelle ledit anticorps est produit par recombinaison.

20. Utilisation selon la revendication 11, dans laquelle ledit anticorps est non purifié.

21. Utilisation selon la revendication 11, dans laquelle ledit anticorps est purifié.
